# EUROPEAN PATENT APPLICATION

(11) **EP 4 219 013 A1**
(43) Date of publication of application: **02.08.2023**
(21) Application number: 21872908.5
(22) Date of filing: 23.09.2021
(51) Int. Cl.: B01L 9/04

(54) **DIAGNOSTIC KIT TRAY**

(30) Priority: 23.09.2020 KR 20200003471 U
(71) Applicant: SD Biosensor, Inc., Gyeonggi-do 16690 (KR)
(72) Inventor: OH, Seong Doo, Hwaseong-si, Gyeonggi-do 18615 (KR); MOON, Seo Hwan, Suwon-si Gyeonggi-do 16313 (KR); CHOI, Byung Kwan, Seongnam-si, Gyeonggi-do 13137 (KR)
(74) Representative: J A Kemp LLP
(86) International application number: PCT/KR2021/012963
(87) International publication number: WO 2022/065880

(57) **Abstract**

A diagnostic kit tray is provided, which includes a tray body including a plurality of receiving recesses for storing diagnostic kits, and a tray cover coupled to an upper portion of the tray body to prevent foreign substances from penetrating into the receiving recesses, in which the tray body includes a lower plate including a plurality of recesses spaced apart from each other, and a locking member positioned at an edge of the lower plate and including a protrusion and a locking jaw to couple with the tray cover, and the tray cover includes a cover plate including a plurality of seating recesses to receive a portion of the diagnostic kit such that the diagnostic kit can be temporarily stored during use, and a coupling portion including a coupling recess to be coupled to the locking member.

## Description

### [TECHNICAL FIELD]

The present disclosure relates to a diagnostic kit tray, and more particularly, to a diagnostic kit tray capable of storing a diagnostic kit capable of diagnosing a disease.

### [BACKGROUND ART]

In recent years, diagnostic kits are widely used for diagnosing human diseases. For the diagnostic kit, a diagnostic method is used which collects blood, urine, or parts from a human body and mixes it with a specific substance for identification. There are various types of diagnostic kits, and diagnostic kits generally have a cylindrical and long shape.

Meanwhile, there is a need for a storage tray that can easily store the diagnostic kit in a sealed state before use. To use the diagnostic kit, the diagnostic kit can be taken out from the tray one by one for use.

However, there are the following problems when using the diagnostic kit in this way.

When using a diagnostic kit, the method of mixing a substance collected from a user with a specific substance requires a certain amount of time until the user can check the result. In addition, the mixing process may also take a long time. In that case, the user may need a separate tray for the diagnostic kit he or she has taken out of the tray, which may cause inconvenience to the user. If there is no separate tray, there may be a problem it that it is difficult to keep the kit standing properly, especially if the bottom of the diagnostic kit has a hemispherical shape.

### [SUMMARY]

The present disclosure is provided to solve one or more problems including those described above, and an object of the present disclosure is to provide a diagnostic kit tray capable of safely storing a diagnostic kit before use, and also temporarily safely storing the diagnostic kit during use.

The present disclosure is to provide a diagnostic kit tray capable of safely storing the diagnostic kit for the purpose of both storing and temporary storing.

The present disclosure is not limited to the above, and other objects that are not mentioned above can be clearly understood by those skilled in the art based on the description provided below.

The present disclosure provides a diagnostic kit tray capable of housing several diagnostic kits or temporarily storing the diagnostic kit during use.

According to an embodiment of the present disclosure, a diagnostic kit tray is provided, which may include a tray body including a plurality of receiving recesses for storing diagnostic kits, and a tray cover coupled to an upper portion of the tray body to prevent foreign substances from penetrating into the receiving recesses, in which the tray body may include a lower plate including a plurality of recesses spaced apart from each other, and a locking member positioned at an edge of the lower plate and including a protrusion and a locking jaw to couple with the tray cover, and the tray cover may include a cover plate including a plurality of seating recesses to receive a portion of the diagnostic kit such that the diagnostic kit can be temporarily stored during use, and a coupling portion including a coupling recess to be coupled to the locking member.

According to an embodiment, the seating recess may include a lower recess portion, and an upper recess portion positioned above the lower recess portion and having a larger cross-sectional area than the lower recess portion, in which a length of the lower recess portion may be provided longer than a length of the upper recess portion, and when the diagnostic kit tray, with the tray body and the tray cover in a state of being coupled to each other, is viewed from above, each one of the seating recesses may be positioned between the receiving recesses.

According to an embodiment, when the diagnostic kit, with the tray body and the tray cover in a state of being coupled to each other, is positioned in the receiving recess, a length between an upper portion of the diagnostic kit and the cover plate may be provided such that they are spaced apart by a predetermined distance, and the predetermined distance may be in the range of 0.1 mm to 1.5 mm.

According to an embodiment, the tray body may include an inner protection part positioned to cover an upper space of the receiving recess and prevent foreign substances from entering the receiving recess, and an inner joining part positioned on a lower surface of the receiving recess and provided to be contacted and coupled to the inner protection part when the diagnostic kit is positioned in the receiving recess, and the tray cover may include an outer protection part positioned to cover an upper space of the seating recess and prevent foreign substances from entering the seating recess, and an outer joining part positioned on a lower surface of the seating recess and provided to be contacted and coupled to the outer protection part when the diagnostic kit is positioned in the seating recess to be temporarily stored during use.

According to an embodiment, the diagnostic kit tray may include an inner contact portion positioned on a bottom surface of the receiving recess, and having magnetism, and an outer contact portion positioned on a bottom surface of the seating recess, and having magnetism, in which the inner contact portion or the outer contact portion may be formed so as to be coupled to or separated from an external contact portion positioned on a lower outer side of the diagnostic kit tray and having magnetism.

The diagnostic kit tray of the present disclosure provides a plurality of receiving recesses and a plurality of seating recesses, so that the diagnostic kits can be stored when not in use and also temporarily stored during use, and user convenience can be improved.

In addition, according to the present disclosure, the tray body and the tray cover are firmly coupled to each other to prevent foreign substances from entering into the receiving recess.

In addition, according to the present disclosure, by providing the inner protection part and the outer protection part, it is possible to prevent foreign substances from entering the receiving recess and the seating recess, and it is also possible to check the used space through a color change.

In addition, according to the present disclosure, by providing the inner contact portion, the outer contact portion, and the external contact portion, the diagnostic kit can be stably supported when received in the receiving recess or the seating recess.

In addition, according to the present disclosure, the tray cover and the tray body are firmly coupled to each other to allow stable movement.

The effects of the present disclosure are not limited to those described above, and effects not mentioned will be clearly understood by those skilled in the art from the description and the accompanying drawings.

### [BRIEF DESCRIPTION OF THE DRAWINGS]

The above and other objects, features and advantages of the present disclosure will be described with reference to the accompanying drawings described below, where similar reference numerals indicate similar elements, but not limited thereto, in which:
FIG. 1 is a perspective view of a diagnostic kit tray according to an embodiment of the present disclosure;
FIG. 2 is an exploded perspective view of the diagnostic kit tray of FIG. 1;
FIG. 3 is a perspective view of the tray body of FIG. 1;
FIG. 4 is a plan view of the tray body of FIG. 1;
FIG. 5 is a cross-sectional view taken along line I-I' in FIG. 4;
FIG. 6 is a plan view of the tray cover of FIG. 1;
FIG. 7 is a cross-sectional view taken along line II-II' of FIG. 6;
FIGS. 8 and 9 are cross-sectional view of a receiving recess according to another embodiment of the present disclosure;
FIGS. 10 and 11 are cross-sectional views of a seating recess according to another embodiment of the present disclosure;
FIGS. 12 and 13 are cross-sectional views of a receiving recess and a seating recess according to another embodiment of the present disclosure; and

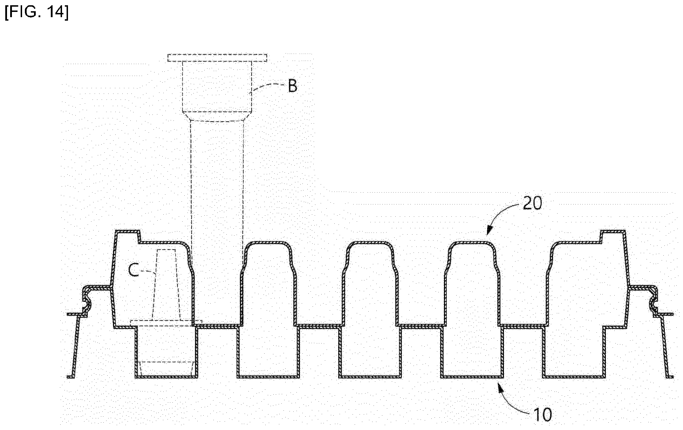

FIG. 14 is a cross-sectional view showing a body and a cap for a diagnostic kit are coupled to each other.

### [DETAILED DESCRIPTION]

Hereinafter, the embodiments of the present disclosure will be described in more detail with reference to the accompanying drawings. The embodiments of the present disclosure may be modified in various forms, and the scope of the present disclosure should not be construed as being limited to the following embodiments. The embodiment is provided to more completely explain the present disclosure to those skilled in the art. Accordingly, the shapes of elements in the drawings may be exaggerated in some parts to emphasize a clearer description. Further, the terms and words used in the present disclosure and claims should not be construed as limited to ordinary or dictionary meanings, but should be interpreted as the meaning and concept in accordance with the principle that the inventor can appropriately define the concept of the term in order to explain the inventive concept in the best way as possible.

The present disclosure provides a diagnostic kit tray 1. A diagnostic kit is provided to mix body tissues, blood, and the like collected from a user therein for the diagnosis of a human disease. It is a medical product that can then detect a specific disease by detecting a predetermined reaction or color change. The diagnostic kit of the present disclosure will be described by referring to a diagnostic kit including a cap (C) and a body (B). The cap and the body of the diagnostic kit will be described based on the example shown in the drawings. However, the shape of the diagnostic kit is not limited thereto and may take various shapes. As shown in the drawing, the diagnostic kit tray 1 of the present disclosure is provided to house diagnostic kits inside or outside.

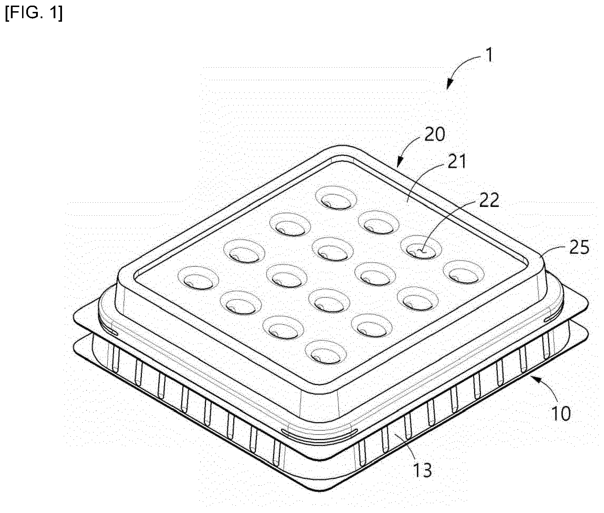

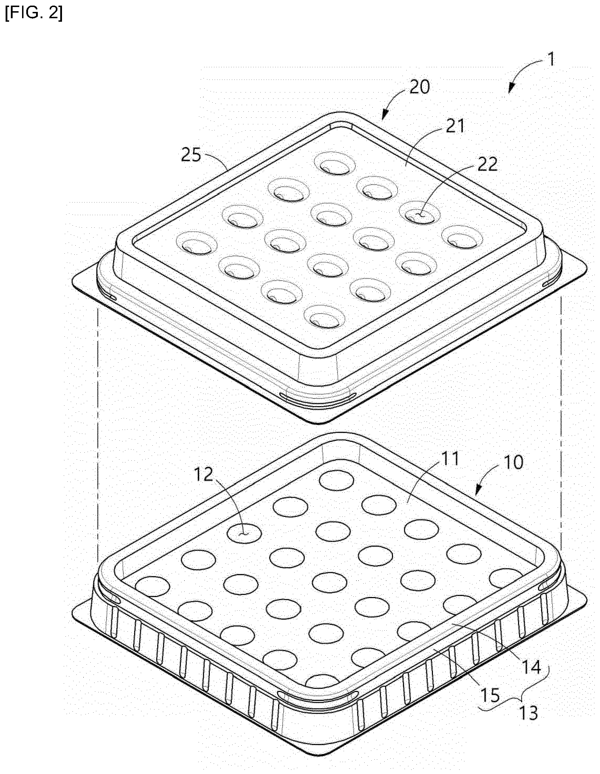

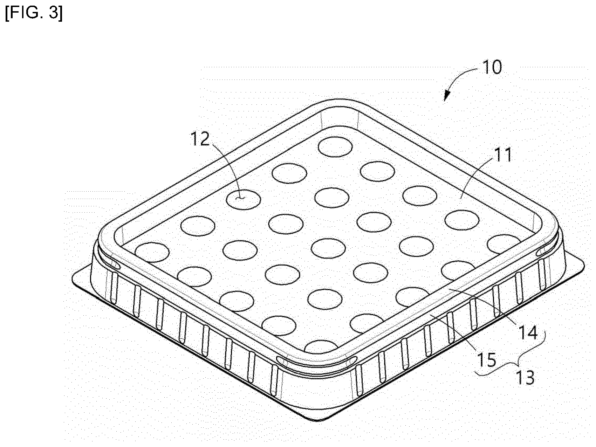

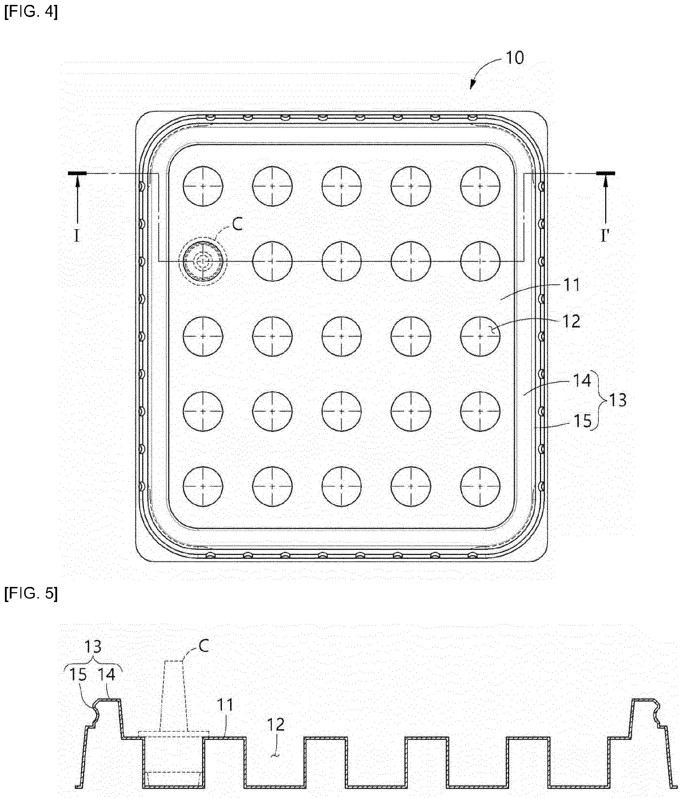

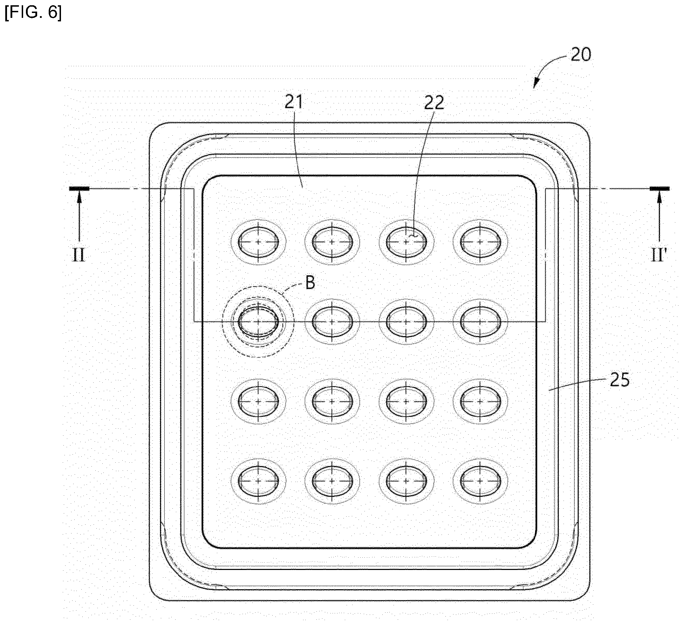

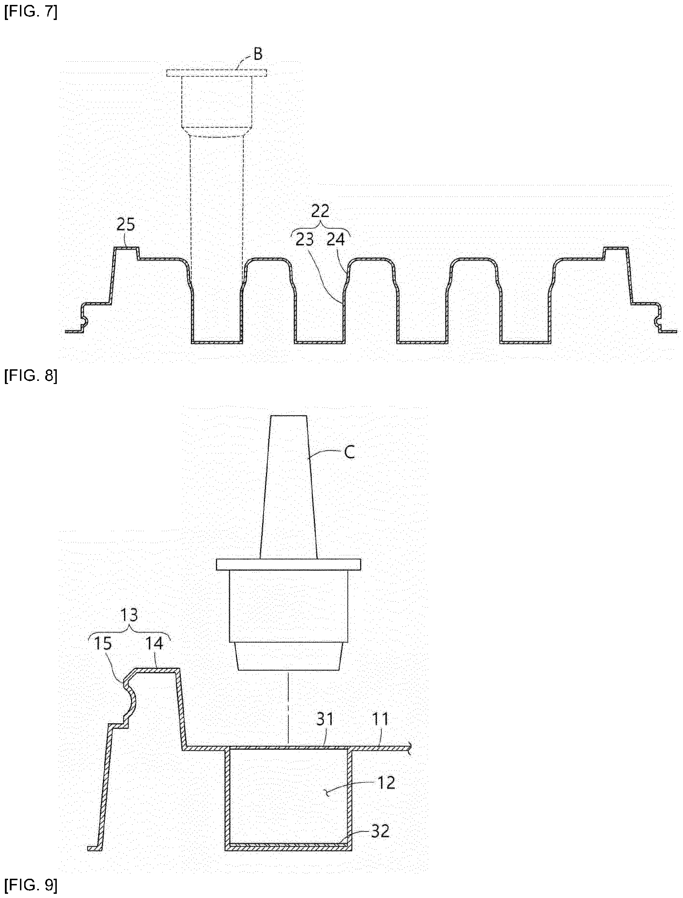

FIG. 1 is a perspective view of the diagnostic kit tray 1 according to an embodiment of the present disclosure, FIG. 2 is an exploded perspective view of the diagnostic kit tray 1 of FIG. 1, FIG. 3 is a perspective view of a tray body 10 of FIG. 1, FIG. 4 is a plan view of the tray body 10 of FIG. 1, FIG. 5 is a cross-sectional view taken along line I-I' in FIG. 4, FIG. 6 is a plan view of a tray cover 20 of FIG. 1, and FIG. 7 is a cross-sectional view taken along line II-II' of FIG. 6.

Referring to FIGS. 1 to 7, the diagnostic kit tray 1 includes a tray body 10 and a tray cover 20. The tray body 10 and the tray cover 20 may be coupled to each other to form one diagnostic kit tray 1. The diagnostic kit tray 1 may be made of a plastic material. For example, it may be made of a PP material. Alternatively, it may be made of various plastic materials.

A plurality of diagnostic kits before use may be stored in the tray body 10. An example will be described herein, in which 25 diagnostic kits can be housed in the tray body 10. However, the number of diagnostic kits that can be housed in the tray body 10 is not limited thereto, and there may be less or more than 10 diagnostic kits.

The tray body 10 includes a lower plate 11, a receiving recess 12, and a locking member 13.

The lower plate 11 may be formed in a substantially rectangular shape. A plurality of receiving recesses 12 may be formed in the lower plate 11. The plurality of receiving recesses 12 may be spaced apart from each other by a predetermined distance. The diagnostic kit may be received in the receiving recess 12. For example, a cap portion of the diagnostic kit may be positioned in the receiving recess 12. The receiving recess 12 may be provided with the same cross-sectional area in an upward or downward direction. All of the plurality of receiving recesses 12 may be provided with the same shape.

The locking member 13 may be formed at an edge of the lower plate 11. The locking member 13 may be positioned to surround the lower plate 11. When the locking member 13 is coupled to a tray cover 20 to be described below, the locking member 13 may be coupled to a coupling portion 25 to couple the tray cover 20 and the tray body 10 to each other.

The locking member 13 includes a protrusion 14 and a locking jaw 15. The protrusion 14 may protrude from the lower plate 11 in a direction opposite to the position of the receiving recess 12. The locking jaw 15 protruding outward may be formed at an outer end of the protrusion 14. The locking jaw 15 is provided so that it can be firmly coupled to the coupling portion 25 to be described below.

The tray cover 20 may be positioned above the tray body 10 when coupled to the tray body 10. The tray cover 20 may be positioned on the upper part of the tray body 10 to prevent foreign substances from entering the receiving recess 12 or to prevent foreign substances from entering the diagnostic kit when the diagnostic kit is positioned in the receiving recess 12.

The tray cover 20 includes a cover plate 21, a seating recess 22, and the coupling portion 25. A plurality of seating recesses 22 may be formed in the cover plate 21. The plurality of seating recesses 22 may be spaced apart from each other by a predetermined distance. In the example of the present disclosure, it is exemplified that 16 seating recesses 22 are provided, but aspects are not limited thereto. When the tray cover 20 and the tray body 10 are coupled to each other, the seating recess 22 may be positioned between the receiving recesses 12 when viewed from above. That is, the seating recess 22 may be positioned between adjacent receiving recesses 12.

The seating recess 22 includes a lower recess portion 23 and an upper recess portion 24. The lower recess portion 23 may be positioned at a lower portion of the seating recess 22. The upper recess portion 24 may be positioned at an upper portion of the lower recess portion 23. The lower recess portion 23 may be provided with the same cross-sectional area in a downward or upward direction. The cross-sectional area of the upper recess portion 24 may be greater than that of the lower recess portion 23. The length of the lower recess portion 23 may be longer than that of the upper recess portion 24. In this example, it is defined that a length in a direction from the tray cover 20 or the tray body 10 toward the tray body 10 or the tray cover 20 is the length.

Since the cross-sectional area of the upper recess portion 24 is greater than that of the lower recess portion 23, the diagnostic kit can be more easily inserted into the seating recess 22 to be stored. In addition, the lower recess portion 23 has a larger cross-sectional area than the upper recess portion 24, and can stably support the body, which is a part of the diagnostic kit, when the body is received therein.

The coupling portion 25 may be coupled to the locking member 13. The coupling portion 25 may be formed around the cover plate 21. The coupling portion 25 may be positioned to surround the cover plate 21. The coupling portion 25 may protrude upward and may have a coupling recess therein. The coupling recess formed inside may be coupled to the protrusion 14 and the locking jaw 15 of the locking member 13 to stably fasten the tray body 10 and the tray cover 20.

When the tray body 10 is coupled to the tray cover 20 with the diagnostic kit being received in the receiving recess 12, the cover plate 21 and the upper portion of the diagnostic kit may be spaced apart by a predetermined distance. For example, the predetermined distance may be in the range of 0.1 mm to 1.5 mm. Preferably, the distance may be 1.1 mm or less.

The diagnostic kit tray 1 described above may prevent foreign substances from entering during storage of the diagnostic kit, and stably support the diagnostic kit during storage or transport of the diagnostic kit. In addition, in the diagnostic kit tray 1, the tray body 10 and the tray cover 20 are firmly coupled to each other such that the cover does not come off easily. In addition, the diagnostic kit tray 1 can be used to temporarily store the diagnostic kit in the seating recess 22 during use of the diagnostic kit, thereby providing convenience to the user.

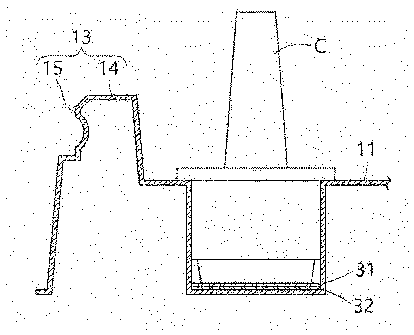

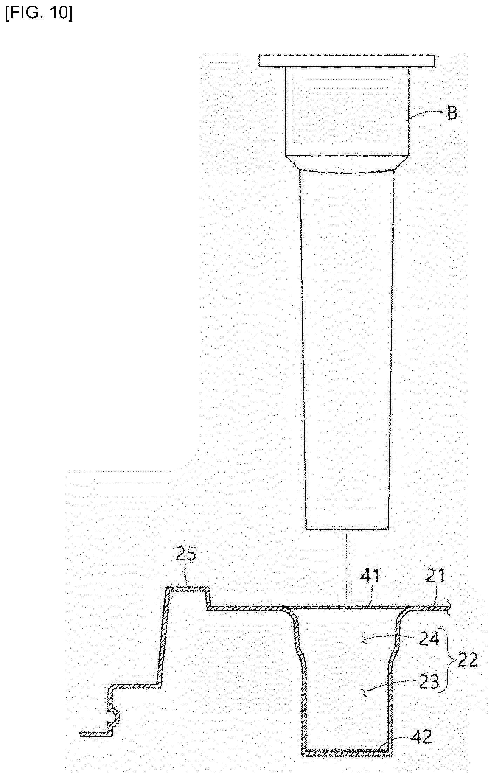

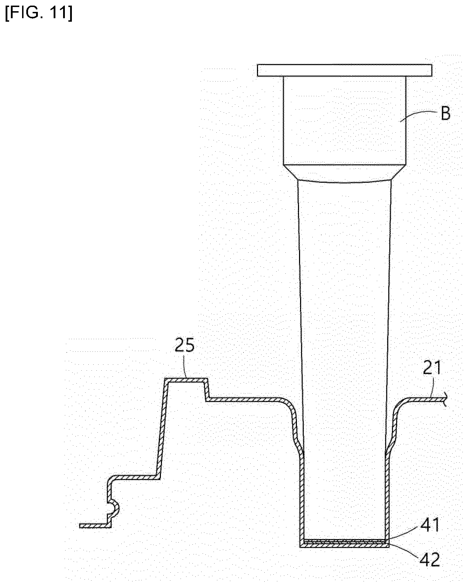

Hereinafter, another embodiment of the present disclosure will be described. FIGS. 8 and 9 are cross-sectional views of the receiving recess 12 according to another embodiment of the present disclosure, and FIGS. 10 and 11 are cross-sectional views of the seating recess 22 according to another embodiment of the present disclosure.

Hereinafter, referring to FIGS. 8 to 11, the diagnostic kit tray 1 may further include an inner protection part 31, an inner joining part 32, an outer protection part 41, and an outer joining part 42.

The inner protection part 31 may be positioned above the receiving recess 12 to prevent foreign substances from entering the receiving recess 12. The inner protection part 31 may be coupled to an upper portion of the receiving recess 12. For example, the inner protection part 31 may be provided in the form of a thin vinyl and in a state of being coupled to the upper portion of the receiving recess 12.

The inner joining part 32 may be positioned on a lower bottom surface of the receiving recess 12. An adhesive material may be partially applied to an upper portion of the inner joining part 32. When the diagnostic kit is housed in the receiving recess 12, the lower portion of the diagnostic kit may push at least the inner protection part 31 and move to the inner joining part 32. In particular, the inner protection part 31 may be moved to the lower portion of the receiving recess 12 together with the diagnostic kit, and then the inner protection part 31 may be coupled to the inner joining part 32. Therefore, when the diagnostic kit exits the receiving recess 12, the inner protection part 31 may remain coupled to the inner joining part 32 and thus is not moved.

The outer joining part 42 is positioned at an upper portion the seating recess 22 to prevent foreign substances from entering the seating recess 22. The outer protection part 41 may be coupled to the upper portion of the seating recess 22. For example, the outer protection part 41 may be provided in the form of a thin vinyl and in a state of being coupled to the upper portion of the seating recess 22.

The outer joining part 42 may be positioned at a lower bottom surface of the seating recess 22. An adhesive material may be partially applied to an upper portion of the outer joining part 42. When the diagnostic kit is received in the seating recess 22, the lower portion of the diagnostic kit may push at least the outer protection part 41 and move to the outer joining part 42. In particular, the outer protection part 41 may be moved to the lower portion of the seating recess 22 together with the diagnostic kit, and then the outer protection part 41 may be coupled to the outer joining part 42. Accordingly, when the diagnostic kit exits the seating recess 22, the outer protection part 41 may remain coupled to the outer joining part 42 and thus is not moved.

There may be a partial change of color when the inner protection part 31 is coupled to the inner joining part 32, or when the outer protection part 41 is coupled to the outer joining part 42. For example, a specific material may be mixed with a lower surface of the inner protection part 31 or outer protection part 41, or with an upper surface of the inner joining part 32 or outer joining part 42 such that a material that can cause a change in color may be provided when the lower surface and the upper surface come into contact with each other, and as a result a change of color may occur. Through this, the user can check the currently used diagnostic kit, and by checking which of the receiving recesses 12 or the seating recesses 22 is actually used, it is possible to ensure stable use of the diagnostic kits.

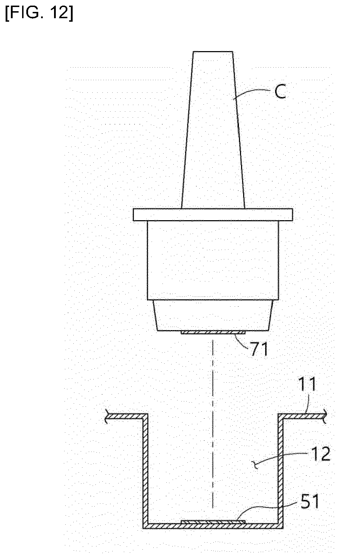

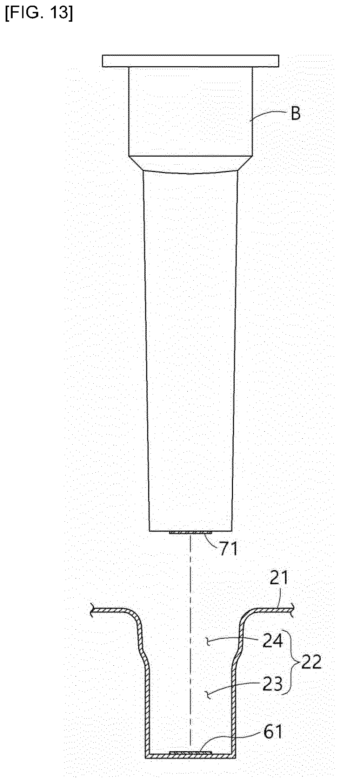

Hereinafter, yet another embodiment of the present disclosure will be described. FIGS. 12 and 13 are views showing cross-sections of the receiving recess 12 and the seating recess 22, according to yet another embodiment of the present disclosure.

The diagnostic kit tray 1 may include an inner contact portion 51, an outer contact portion 61, and an external contact portion 71 positioned at a lower portion of the diagnostic kit. The inner contact portion 51 may be positioned on the bottom surface of the receiving recess 12. The outer contact portion 61 may be positioned on the bottom surface of the seating recess 22. The external contact portion 71 may be positioned on the lower surface of the diagnostic kit. The inner contact portion 51, the outer contact portion 61, and the external contact portion 71 may each be made of a material having magnetism. Through this, when the diagnostic kit is positioned in the receiving recess 12, the inner contact portion 51 and the external contact portion 71 can be firmly coupled to each other. In addition, when the diagnostic kit is positioned in the seating recess 22, the outer contact portion 61 and the external contact portion 71 may be firmly coupled to each other.

As described above, the diagnostic kit tray of the present disclosure provides a plurality of receiving recesses and a plurality of seating recesses, so that the diagnostic kits can be stored when not in use and also temporarily stored during use, and user convenience can be improved.

In addition, according to the present disclosure, the tray body and the tray cover are firmly coupled to each other to prevent foreign substances from entering into the receiving recess.

In addition, according to the present disclosure, by providing the inner protection part and the outer protection part, it is possible to prevent foreign substances from entering the receiving recess and the seating recess, and it is also possible to check the used space through a color change.

In addition, according to the present disclosure, by providing the inner contact portion, the outer contact portion, and the external contact portion, the diagnostic kit can be stably supported when received in the receiving recess or the seating recess.

In addition, according to the present disclosure, the tray cover and the tray body are firmly coupled to each other to allow stable movement.

The above detailed description is illustrative of the present disclosure. In addition, the foregoing is intended to illustrate and describe preferred embodiments of the present disclosure, and the present disclosure may be used in various other combinations, modifications, and environments. That is, changes or modifications are possible within the scope of the concept of the invention disclosed in this specification, and within the scope equivalent to the written disclosure and/or within the scope of skill or knowledge in the art. The foregoing embodiment describes the best state for implementing the technical spirit of the present disclosure, and various changes required in specific application fields and uses of the present disclosure are also possible. Therefore, the above detailed description of the invention is not intended to limit the present disclosure to the disclosed embodiments. In addition, the appended claims should be construed to cover other embodiments as well.

## Claims

1. A diagnostic kit tray comprising:
a tray body including a plurality of receiving recesses for storing diagnostic kits; and
a tray cover coupled to an upper portion of the tray body to prevent foreign substances from penetrating into the receiving recesses,
wherein the tray body includes:
a lower plate including a plurality of recesses spaced evenly apart from each other; and
a locking member positioned at an edge of the lower plate and including a protrusion and a locking jaw to couple with the tray cover, and
the tray cover includes:
a cover plate including a plurality of seating recesses to receive a portion of the diagnostic kit such that the diagnostic kit can be temporarily stored during use; and
a coupling portion including a coupling recess to be coupled to the locking member.

2. The diagnostic kit tray according to claim 1, wherein the seating recess includes:
a lower recess portion; and
an upper recess portion positioned above the lower recess portion and having a larger cross-sectional area than the lower recess portion,
wherein a length of the lower recess portion is provided longer than a length of the upper recess portion, and
when the diagnostic kit tray, with the tray body and the tray cover in a state of being coupled to each other, is viewed from above, each one of the seating recesses is positioned between the receiving recesses.

3. The diagnostic kit tray according to claim 2, wherein, when the diagnostic kit, with the tray body and the tray cover in a state of being coupled to each other, is positioned in the receiving recess, a length between an upper portion of the diagnostic kit and the cover plate is provided such that they are spaced apart by a predetermined distance, and
the predetermined distance is in a range of 0.1 mm to 1.5 mm.

4. The diagnostic kit tray according to any one of claims 1 to 3, wherein the tray body includes:
an inner protection part positioned to cover an upper space of the receiving recess and prevent foreign substances from entering the receiving recess; and
an inner joining part positioned on a lower surface of the receiving recess and provided to be contacted and coupled to the inner protection part when the diagnostic kit is positioned in the receiving recess, and
the tray cover includes:
an outer protection part positioned to cover an upper space of the seating recess and prevent foreign substances from entering the seating recess; and
an outer joining part positioned on a lower surface of the seating recess and provided to be contacted and coupled to the outer protection part when the diagnostic kit is positioned in the seating recess to be temporarily stored during use.

5. The diagnostic kit tray according to any one of claims 1 to 3, comprising:
an inner contact portion positioned on a bottom surface of the receiving recess, and having magnetism; and
an outer contact portion positioned on a bottom surface of the seating recess, and having magnetism,
wherein the inner contact portion or the outer contact portion is formed so as to be coupled to or separated from an external contact portion positioned on a lower outer side of the diagnostic kit tray and having magnetism.
